# EUROPÄISCHE PATENTSCHRIFT

(11) **EP 2 448 503 B1**
(45) Veröffentlichungstag und Bekanntmachung des Hinweises auf die Patenterteilung: **25.12.2013**
(21) Anmeldenummer: 10724540.9
(22) Anmeldetag: 23.06.2010
(51) Int. Cl.: A61B 17/3213, A61F 9/013, A61B 17/00, A61F 9/007

(54) **SKALPELL, INSBESONDERE FÜR OPHTHALMOLOGISCHE ANWENDUNGEN**
SCALPEL, IN PARTICULAR FOR OPHTHALMOLOGIC APPLICATIONS
SCALPEL, NOTAMMENT POUR APPLICATIONS OPHTALMOLOGIQUES

(30) Priorität: 29.06.2009 DE 102009030874
(43) Veröffentlichungstag der Anmeldung: 09.05.2012
(73) Patentinhaber: Rheinische Fachhochschule Köln GgmbH, 50676 Köln (DE)
(72) Erfinder: SAXLER, Wilfried, 50321 Brühl (DE); THIMM, Benedikt, 50823 Köln (DE)
(74) Vertreter: Jostarndt, Hans-Dieter
(86) Internationale Anmeldenummer: PCT/EP2010/058896
(87) Internationale Veröffentlichungsnummer: WO 2011/000752

(56) Entgegenhaltungen:
- US-A1- 2004 182 823
- US-A1- 2004 215 174
- US-A1- 2005 245 953

## Beschreibung

Die Erfindung betrifft ein Skalpell, insbesondere für ophthalmologische Anwendungen.

Aus der Druckschrift DE 19652098 C2 ist ein chirurgisches Instrument bekannt, welches gänzlich (also Griff und Klinge) aus keramischem Werkstoff besteht und im Spritzgießverfahren hergestellt ist. Ein anderes Skalpell besteht aus einer Klinge aus Keramik mit Schneide und Stiel, wobei der Stiel unlösbar im distalen Ende eines Schaftes aus Edelstahl steckt (DE 29919914 U1). Solche Skalpelle werden meistens zur einmaligen Verwendung eingesetzt.

Mit der US 5 718 708 A ist ein ophthalmologisches Instrument veröffentlicht, welches als Schäl- und Schabwerkzeug ausgebildet ist. Das Werkzeug hat einen stumpf auslaufenden Kopf, an der seitliche drei Schneiden angeordnet sind. Der Vorderteil des Werkzeugkopfs ist gegen das Mittelteil mit einem kleinen Winkel abgeknickt. Die Unterseite des abgeknickten Teils trägt eine raue Struktur zum Schaben. Das Instrument ist für flache Schnitte oder flache Einstiche bei Kataraktoperationen nicht zu gebrauchen. Ein für flache Schnitte oder Einstiche geschaffenes Skalpell findet sich in der US 5 098 438 A.

Die US 3 325 900 A zeigt ein Skalpell aus einem metallischen Griff und einem metallischen Werkzeugkopf. Im Werkzeugkopf ist ein Klingenhalter ausgebildet, in den ein- oder zweischneidige metallische Klingen einsetzbar sind.

Es ist ein anderes chirurgisches Instrument bekannt, welches mit einer zur Skalpellspitze hin verschiebbaren Schutzhülse ausgestattet ist (US 2004-0215174 A1). Das Instrument ist ein Hightech-Werkzeug und demnach aufwändig ausgebildet und besteht aus einer Vielzahl von Einzelteilen. Die Skalpellklinge ist lösbar befestigt, so dass sie bei Verschleiß ausgewechselt werden kann.

Dokument US2005/245953 A1 offenbart ein Skalpell nach dem Oberbegriff des Anspruchs 1.

Der Erfindung liegt die Aufgabe zugrunde, ein einfach aufgebautes, mehrfach verwendbares und preisgünstiges Skalpell insbesondere für ophthalmologische Anwendungen anzugeben, mit dem im Besonderen flache Schnitte oder flache Stechbewegungen ausführbar sind.

Die Aufgabe wird erfindungsgemäß durch die Merkmale des unabhängigen Anspruches gelöst, während den abhängigen Ansprüchen vorteilhafte Weiterbildungen der Erfindung zu entnehmen sind.

Der Kern der Erfindung liegt darin, dass am Griffkopf keil- oder dachkantenartig zwei gegeneinander geneigte Flächen ausgebildet sind und in einer ersten der geneigten Flächen eine Öffnung zum Einsetzen der Klinge vorhanden ist, wobei die eingesetzte Klinge derart in der Öffnung zu liegen kommt, dass die Rückseitenfläche des aus Klingenschaft und Klingenblatt ausgebildeten Körpers bündig ist mit der ersten geneigten Fläche.

Durch die bündige Anordnung von Rückseitenfläche mit der ersten geneigten Fläche können auf der Hornhaut des Auges flache Einschnitte oder flache Stechbewegungen ausgeführt werden, die insbesondere bei der Operation an der Augenlinse notwendig sind.

Weitere vorzugsweise Ausgestaltungen und Ergänzungen werden im Folgenden angesprochen, wobei die Merkmale einzeln oder miteinander gemeinsam verwirklicht sein können.

Die Öffnung (Bohrung) im Griffkopf ist passend zum Querschnitt des Klingenschafts ausgebildet. Das heißt, dass angepasst an die Ausbildung des Klingenschafts die Öffnung im Querschnitt entsprechend ausgebildet ist.

Vorzugsweise ist der Klingenschaft als ein Körper ausgebildet, der einen parallel zur Längsachse geschnittenen Zylinder darstellt, wobei die Schnittebene des Körpers in der Längsachse des Zylinders (Zylinderachse) liegt. Die Rückseitenfläche entspricht der Ebene des halbierten Zylinders. Eine (halb-)zylindrische Ausbildung erlaubt eine genaue Zentrierung der Klinge im Griff. Des weiteren wird das Einsetzen des Klingenschafts und das Befestigen (in einem Klebevorgang) vereinfacht, da der Kleber sich gleichmäßig auf der zylindrischen Oberfläche verteilt; es entstehen keine Ecken.

Ein wesentlicher Punkt ist, dass die Länge der Klinge so bemessen ist, dass die Klinge innerhalb einer gedachten, über das Ende des Griffkopfs hinausragenden Umhüllung verbleibt. Die Länge der Klinge steht also an keinem Punkt über der am Ende des Griffkopfs hinausragenden (gedachten) Umhüllung über. Dies hat den Vorteil, dass die Klinge eines auf einem Tablett liegenden Skalpells nicht die Unterlage berührt. Es kann somit nicht zu einer Kontamination oder einer Beschädigung durch die Berührung mit der Unterlage kommen.

Die erste geneigte Fläche am Griffkopf sollte gegen die Längsachse des Griffs mit einem Winkel zwischen 25° und 35° geneigt sein, vorzugsweise mit einem Winkel von 30°.

Bei der 'Normalausführung' des Skalpells sind an der Klinge zwei Schneiden ausgebildet. Bei einer solchen Ausführung laufen die beiden Schneiden der Klinge symmetrisch zur Längsachse der Klinge in Form eines gleichschenkligen Dreiecks zusammen. Die Form des gleichschenkligen Dreiecks würde in der symmetrischen Ausführung in einem Winkel von 45° auslaufen.

Die Schneidkanten der Klinge sind von der Rückseitenfläche her zur Klingenoberseite angeschrägt. Es sind besonders zwei Ausführungen untersucht worden. Bei einer ersten beträgt der Schneidkantenwinkel ca. 45°; in einer zweiten Klingenform hat der Winkel den Wert von ca. 30°. Die Ausbildungen sind in der Figurenbeschreibung näher erläutert.

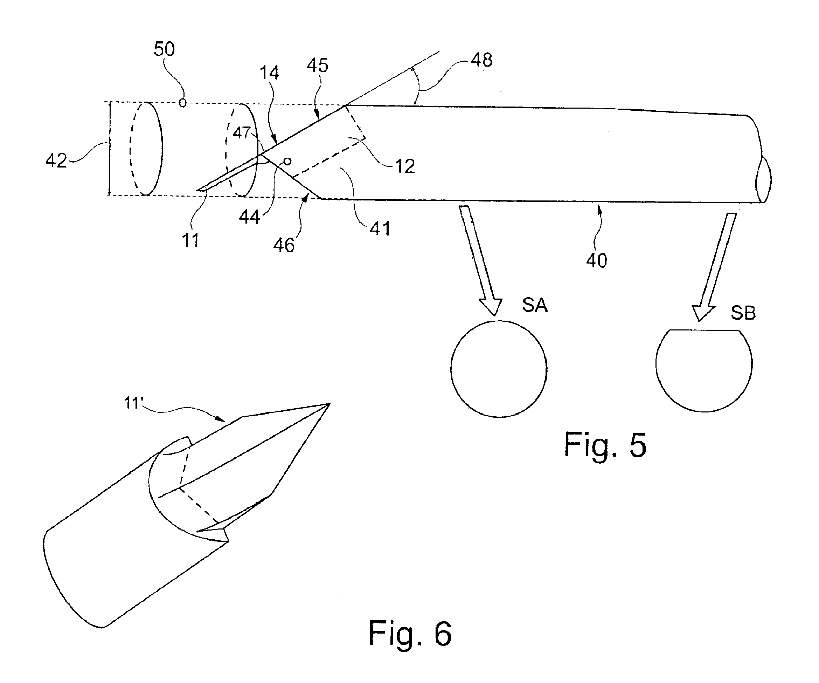

Ein Schneidkantenwinkel von ca. 45° wird hergestellt bei Klingen, die eine bestimmte Dicke haben. Wird der Schneidkantenwinkel verringert, beispielsweise auf 30°, dann entsteht eine sehr flache Ausbildung der Schneide. Oberseite und Rückseite der Klinge sind nicht mehr parallel. Bei dieser Ausbildung hat die Klinge keine konstante Schneidendicke, da die Kanten auf den Schneiden zueinander hinlaufen und eine gemeinsame, in der Mitte der Klinge liegende 'Mittellinie' bilden (siehe Fig. 6).

Die Klinge besteht aus Keramik (Zirkonoxid, Aluminiumoxid oder Mischoxid=Keramik) und wird durch Schleif- und Polierprozesse bearbeitet. Zirkonoxid hat sich als besonders geeignet herausgestellt. Der keramische Ausgangswerkstoff wird in einem HIP-Vorgang (heißisostatisches Pressen) behandelt und ist danach porenfrei, wodurch er sich sehr gut sterilisieren lässt. Skalpelle aus Zirkonoxid vereinen hohe Härte und Verschleißfestigkeit mit geringerer Stoßempfindlichkeit.

Andere Klingenwerkstoffe sind für die Herstellung des Skalpells natürlich ebenso geeignet, wobei auch Diamant infrage kommt. Diamant kann aufgrund seiner hohen Härte und der daraus resultierenden Verschleißfestigkeit mehrmals eingesetzt werden. Allerdings ist Diamant sehr stoßempfindlich und zusätzlich sehr teuer.

Metall-Klingen sind nur beschränkt geeignet, da sie nach einmaligem Gebrauch nicht mehr verwendbar sind.

Die Klinge ist an ihrem Schaft in den Griffkopf unverlierbar eingeklebt. Durch den satten Sitz des Klingenschafts in der Öffnung im Griffkopf genügt ein Tropfen Sekundenkleber, um den Sitz und die Festigkeit herzustellen. Das so hergestellte Skalpell ist mehrfach sterilisierbar und damit mehrfach verwendbar.

Der Durchmesser des (halb-)zylindrischen Klingenschafts ist beispielsweise 4 mm. Die Dicke der Schneide: 0,2 bis 0,3 mm; die Schneidkanten sind gegen die Rückseitenfläche mit einem Winkel von ca. 45° angeschrägt. Bei Einsatz präzise arbeitender Schleifmaschinen entstehen Flächen mit hoher Glätte, so dass die Flächen nicht notwendigerweise weiter poliert werden müssen. Allerdings kann sich nach dem Schleifvorgang noch ein Polierschritt für die Schneidenflächen anschließen mit Diamantschleifscheiben mit Körnungen im µm-Bereich.

Die vorliegende Erfindung ist nicht auf die vorstehend beschriebenen Ausführungsformen beschränkt, sondern umfasst auch alle im Sinne der Erfindung gleichwirkenden Ausführungsformen. Skalpelle mit nur einer Schneide an der Klinge sollen ebenso erfasst sein. Weiterhin lässt sich die Erfindung beispielsweise grundsätzlich auch auf alle mikrochirurgischen Skalpelle anwenden.

Weitere Einzelheiten und Vorteile der Erfindung werden Ausführungsbeispielen anhand von Figuren erläutert. Sie zeigen im Einzelnen
Figur 1: eine perspektivische Darstellung einer ersten Klinge;
Figur 2: ein Seitenansicht einer Klinge;
Figur 3: eine erste perspektivische Darstellung Klinge im Griffkopf;
Figur 4: eine zweite perspektivische Darstellung Klinge im Griffkopf;
Figur 5: ein Seitenansicht der Klinge in Griffkopf und
Figur 6: eine zweite Klingenblattform.

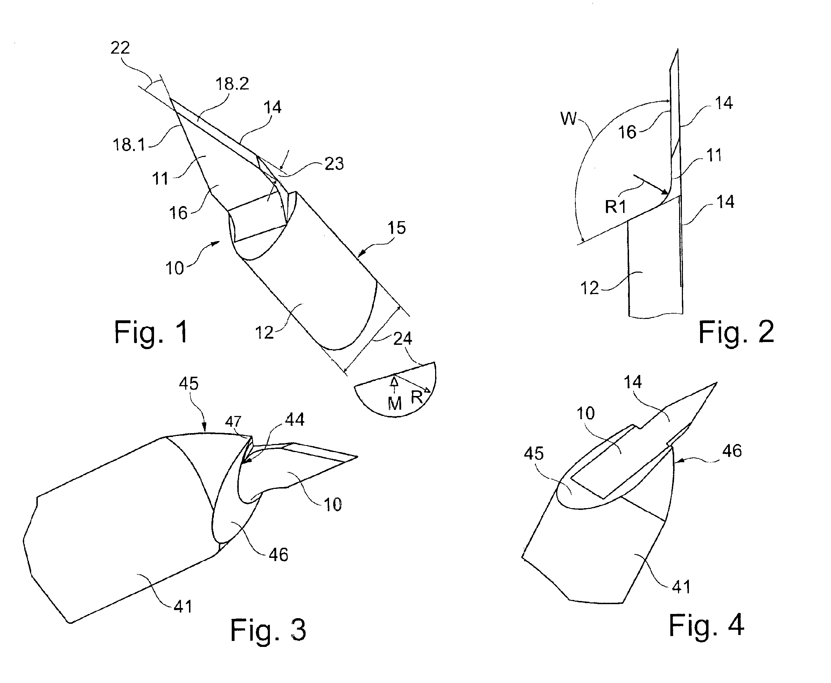

Die Fig. 1 zeigt eine perspektivische Darstellung und Fig. 2 eine Seitenansicht einer Klinge 12, an der ein Klingenschaft 12 und ein Klingenblatt 11 mit zwei Schneiden 18.1, 18.2 vorhanden sind. Die beiden Schneiden der Klinge laufen symmetrisch zur Längsachse der Klinge in Form eines gleichschenkligen Dreiecks zusammen und laufen in einem Winkel 22 von 45° aus. Die Schneiden 18.1, 18.2 sind von der Rückseite 14 her nach vorn (zur Klingenoberseite 16) angeschrägt. Der Klingenschaft 12 und das Klingenblatt 11 bilden gemeinsam einen Körper. Der Körper hat von der Spitze des Klingenblattes bis zum Ende des Klingenschafts eine durchgehende, ebene Rückseitenfläche 14. Der Rückseitenfläche gegenüberliegend ist der Klingenschaft in einer zylindrischen Fläche und das Klingenblatt mit seiner Klingenoberseite 16 ausgebildet.

Die Klingenoberseite 16 der Klinge 11 - in der in Fig. 1 gezeigten Ausführungsform - liegt parallel zur Rückseite 14 der Klinge. Der Klingenschaft 12 ist als ein in seiner Längsachse geschnittener Zylinder mit einem Radius R (bzw. mit einem Zylinder-Durchmesser 24) ausgebildet. Die Zylinderachse (Bezugszeichen M) liegt in der Schnittebene.

Die Rückseitenfläche 14 liegt in der Ebene des halbierten Zylinders. Die Rückseite 15 des Klingenschafts und die Rückseite 14 der Klinge bilden eine gemeinsame Ebene. Die halbzylindrische Ausbildung erlaubt eine genaue Zentrierung der Klinge im Griff 40 (siehe Fig. 3 und 4). Die Flächen der Klinge 10 werden durch Schleifen aus keramischem Werkstoff herausgebildet, wodurch - ausgehend von der Kontur der Schleifscheiben - Rundungen entstehen, beispielsweise ein Übergang vom Klingenschaft zur Klinge, der in den Fig. 1 und 2 in einem stumpfen Winkel W verläuft. Der Scheitel des Übergangs hat - gemäß Fig. 2 - eine Rundung mit einem Radius R1. Die am Übergang von Klinge zum Schaft vorhandenen Rundungen haben einen Radius, der durch die Ausbildung der benutzten Schleifscheibe gegeben ist. Es sind Rundungen mit Radien im Bereich von ca. 1 mm sinnvoll, um Kerbspannungen am Übergang gering zu halten.

Fig. 3 und Fig. 4 zeigen den Griffkopf 41, an dem keilartig oder dachkantenartig zwei gegeneinander geneigte Flächen 45, 46 ausgebildet sind. Die Flächen 45, 46 stoßen dachkantenartig in einer senkrecht zur Achse des Griffs liegenden Kante (47) zusammen. In der ersten geneigte Fläche 45 ist eine Öffnung 44 (siehe Fig. 5) zum Einsetzen des Klingenschafts 12 vorhanden. Insbesondere zeigt Fig. 4, dass die eingesetzte Klinge 10 derart in der Öffnung 44 zu liegen kommt, dass die Rückseitenfläche 14 bündig mit der ersten geneigten Fläche 45 ist.

Bei ophthalmologischen Operationen, insbesondere bei einem sogenannten Tunnelschnitt an der Augenlinse wird das Skalpell nach der Erfindung so gehalten, dass die Rückseitenfläche etwa parallel (oder wenig geneigt) zur Hornhaut zu liegen kommt. Die Spitze der Klinge kann sodann in voller Länge in die Hornhaut eingeführt und vorgetrieben werden, ohne dass der Griffkopf störend die Hornhaut berührt.

In der Fig. 5 wird der Griff 40 an seinem vorderen Ende in einer Seitenansicht gezeigt. In der Öffnung 44, die die Form des Klingenschafts 12 (nach Fig. 1 halbzylindrisch) hat, ist die Klinge 10 unverlierbar eingeklebt. Die Länge der Klinge 10 ist so bemessen, dass die Klinge 10, genauer gesagt, die Spitze der Klinge innerhalb einer gedachten, über das Ende des Griffkopfs 41 hinausragenden Umhüllung 50 verbleibt. Die Rückseitenfläche 14 liegt in einem Winkel 48 geneigt gegen die Achse des Griffs. Der Winkel 48 kann zwischen 25° und 35° liegen, vorzugsweise soll er 30° betragen.

Als typische Dimensionen des Griffs können genannt werden: Länge des Griffs: 130 mm und Durchmesser 42 des Griffs am Griffkopf 41: 7 bis 8 mm. Am Griffkopf ist der Griff zylindrisch ausgebildet, was durch den Schnitt SA in Fig. 5 angedeutet ist. Zum Ende des Griffs ist eine leichte Abflachung des Zylinders ausgebildet, was den Vorteil hat, dass das Skalpell bei Auflegen auf eine Unterlage in eine eindeutige Position rollen kann. Die Abflachung des Griffs geht von der Zylinderform über in den abgeflachten Teil etwa ab einem Abstand vom Griffkopf von einem Fünftel bis einem Sechstel der Länge des Griffs. Bei einer Länge des Griffs von 130 mm, somit bei etwa 105 mm vom Ende des Griffs. Die Form der Abflachung wird durch den Schnitt SB in Fig. 5 angedeutet. Vorzugsweise ist das Material des Griffs eine Titan-Legierung.

In Fig. 6 wird eine zweite Ausbildung der Klingenoberseite (16) mit den Schneiden der Klinge 11' gezeigt. Diese Ausbildung hat keine konstante Schneidendicke. Die Kante, an der die Schneiden zueinander hinlaufen, erstreckt sich über die gesamte Länge der Klinge und läuft am Klingenschaft aus. Die in Fig. 6 gezeigte Klinge 11' ist im Material stärker ausgebildet, was diese Klinge bruchsicherer macht. Obwohl in Fig. 6 dies nicht besonders deutlich wird, laufen die Flächen der Schneiden am Übergang von Klinge 11' zum Klingenschaft (vergleichbar mit Fig. 2) gerundet aus. Die Rundungen haben einen Radius, der von der Kontur der benutzten Schleifscheibe vorgegeben ist.

### Bezugszeichenliste:

- 10, 10': Klinge
- 11 11': Klingenblatt, zweite Blattform
- 12: Klingenschaft
- 14: Rückseitenfläche
- 15: Rückseite Klingenschaft
- 16: Klingenoberseite
- 18.1 18.2: linke, rechte Schneide
- 20: Winkel Anschrägung
- 22: Winkel gleichschenkliges Dreieck
- 24: Durchmesser
- 25: Zylinderachse
- M: Mittelpunkt Zylinder
- R: Radius Klingenschaft
- R1: Rundung
- W: Winkel
- 40: Griff
- 41: Griffkopf
- 42: Durchmesser (Umhüllung) am Griffkopf
- 44: Öffnung, Bohrung (rund oder quadratisch)
- 45: erste geneigte Fläche (Keil)
- 46: zweite geneigte Fläche
- 47: Kante
- 49: Abflachung
- 48: Winkel Neigung erste Fläche zur Achse Griff
- 50: Umhüllung
- SA: Schnitt nähe Griffkopf
- SB: Schnitt nähe Griffende (mit Abflachung)

## Patentansprüche

1. Skalpell, insbesondere für ophthalmologische Anwendungen, umfassend einen Griff und eine Klinge, wobei in einem Griffkopf (41) des Griffs (40) die aus einem Klingenschaft (12) und einem Klingenblatt (11, 11') bestehende Klinge (10) eingesetzt ist, und am Klingenblatt (11,11') mindestens eine Schneide (18.1, 18.2) ausgebildet ist, wobei
• der Klingenschaft (12) gemeinsam mit dem Klingenblatt (11, 11') als ein Körper ausgebildet ist,
**dadurch gekennzeichnet, dass**
an dem Körper eine durchgehende ebene Rückseitenfläche (14) ausgebildet ist,
• wobei die der Rückseitenfläche (14) gegenüberliegende Vorderseite des Körpers im Bereich des Klingenschafts (12) eine zylindrische Fläche und im Bereich des Klingenblatts (11, 11') die Klingenoberseite bildet, und
• der Griffkopf (41) an seinem Ende zwei gegeneinander geneigte Flächen (45, 46) hat, die dachkantenartig In einer senkrecht zur Achse des Griffs (40) liegenden Kante (47) zusammenstoßen, und
• wobei in einer ersten der geneigten Flächen (45) eine Öffnung (44) zum Einsetzen des Klingenschafts (10, 12) vorhanden ist, und die eingesetzte Klinge (10) derart in der Öffnung (44) zu liegen kommt, dass die ebene Rückseitenfläche (14) bündig ist mit der ersten geneigten Fläche (45).

2. Skalpell nach Anspruch 1, **dadurch gekennzeichnet, dass** der Klingenschaft (12) als ein Körper ausgebildet ist, der einen parallel zur Längsachse geschnittenen Zylinder (M, R) darstellt, welcher seine Schnittebene in der Längsachse des Zylinders hat.

3. Skalpell nach einem der vorhergehenden Ansprüche, **dadurch gekennzeichnet, dass** die Länge der Klinge (10) so bemessen ist, dass die Klinge (10) innerhalb einer gedachten, über das Ende des Griffkopfs (41) hinausragenden Umhüllung (50) verbleibt.

4. Skalpell nach einem der vorhergehenden Ansprüche, **dadurch gekennzeichnet, dass** die erste geneigte Fläche (45) am Griffkopf (41) gegen die Längsachse des Griffs (40) mit einem Winkel (48) zwischen 25° und 35° geneigt ist, vorzugsweise mit einem Winkel von 30°.

5. Skalpell nach einem der vorstehenden Ansprüche, **dadurch gekennzeichnet, dass** an der Klinge (10) zwei Schneiden (18.1, 18.2) ausgebildet sind.

6. Skalpell nach vorstehendem Anspruch, **dadurch gekennzeichnet, dass** die Schneiden (18.1, 18.2) der Klinge (10) symmetrisch zur Längsachse der Klinge (10) in Form eines gleichschenkligen Dreiecks (22) zusammenlaufen.

7. Skalpell nach vorstehendem Anspruch, **dadurch gekennzeichnet, dass** die Form des gleichschenkligen Dreiecks in einem Winkel (22) von 45° ausläuft.

8. Skalpell nach vorstehendem Anspruch, **dadurch gekennzeichnet, dass** die Schneidkanten (18.1, 18.2) von der Rückseitenfläche (14) her zur Klingenoberseite (16) angeschrägt sind.

9. Skalpell nach einem der vorhergehenden Ansprüche, **dadurch gekennzeichnet, dass** die Klinge (12) aus Keramik besteht.

10. Skalpell nach einem der vorhergehenden Ansprüche, **dadurch gekennzeichnet, dass** die Klinge (12) in den Griffkopf (41) eingeklebt ist.

## Claims

1. A scalpel, in particular for ophthalmologic applications, comprising a handle head and a blade, whereby the blade (10), which consists of a blade shank (12) and a blade face (11, 11'), is inserted into a handle head (41) of the handle (40), and at least one cutting edge (18.1, 18.2) is formed on the blade face (11, 11'),
wherein
• the blade shank (12) is configured together with the blade face (11, 11') as a body, on which body a continuous smooth rear surface (14) is formed,
• whereby the front of the body opposite from the rear surface (14) forms a cylindrical surface in the area of the blade shank (12) and forms the top of the blade in the area of the blade face (11, 11'), and
• the end of the handle head (41) has two surfaces (45, 46) that are slanted with respect to each other and that abut, like a roof edge, in an edge (47) that is perpendicular to the axis of the handle (40), and
• whereby, in a first of the slanted surfaces (45), there is an opening (44) for inserting the blade (10, 12), whereby the inserted blade (10) comes to lie in the opening (44) in such a way that the smooth rear surface (14) is flush with the first slanted surface (45).

2. The scalpel according to claim 1, **characterized in that** the blade shank (12) is configured as a body in the form of a cylinder (M, R) that is bisected parallel to the longitudinal axis, which has its intersecting plane in the longitudinal axis of the cylinder.

3. The scalpel according to one of the preceding claims, **characterized in that** the length of the blade (10) is dimensioned in such a way that the blade (10) remains within an imaginary envelope (50) that extends beyond the end of the handle head (41).

4. The scalpel according to one of the preceding claims, **characterized in that** the first slanted surface (45) on the handle head (41) is slanted by an angle (48) between 25° and 35°, preferably by an angle of 30°, relative to the longitudinal axis of the handle (40).

5. The scalpel according to one of the preceding claims, **characterized in that** two cutting edges (18.1, 18.2) are formed on the blade (10).

6. The scalpel according to the preceding claim, **characterized in that** the cutting edges (18.1, 18.2) of the blade (10) converge in the form of an equilateral triangle (22) symmetrically relative to the longitudinal axis of the blade (10).

7. The scalpel according to the preceding claim, **characterized in that** the shape of the equilateral triangle tapers at an angle (22) of 45°.

8. The scalpel according to the preceding claim, **characterized in that** the cutting edges (18.1, 18.2) are beveled from the rear surface (14) towards the top (16) of the blade.

9. The scalpel according to one of the preceding claims, **characterized in that** the blade (12) is made of ceramic.

10. The scalpel according to one of the preceding claims, **characterized in that** the blade (12) is glued into the handle had (41).

## Revendications

1. Scalpel, notamment pour applications ophtalmologiques, comprenant un manche et une lame, dans lequel la lame (10) se composant d'un arbre de lame (12) et d'une partie tranchante de lame (11, 11') est insérée dans une tête de manche (41) du manche (40), et au moins un tranchant (18.1, 18.2) est réalisé sur la partie tranchante de lame (11, 11'),
dans lequel
• l'arbre de lame (12) est réalisé avec la partie tranchante de lame (11, 11') en tant que corps, **caractérisé en ce qu'**une face arrière plane continue (14) est réalisée sur le corps,
• dans lequel le côté avant opposé à la face arrière (14) du corps forme dans la région de l'arbre de lame (12) une face cylindrique et forme dans la région de la partie tranchante de lame (11, 11') le côté supérieur de lame, et
• la tête de manche (41) a deux faces inclinées l'une contre l'autre (45, 46) à son extrémité qui se rencontrent à la manière d'une arête de toit dans une arête (47) située perpendiculairement à l'axe du manche (40), et
• dans lequel une ouverture (44) pour l'insertion de l'arbre de lame (10, 12) est présente dans une première des faces inclinées (45), et la lame insérée (10) vient en appui dans l'ouverture (44) de telle sorte que la face arrière plane (14) est à fleur avec la première face inclinée (45).

2. Scalpel selon la revendication 1, **caractérisé en ce que** l'arbre de lame (12) est réalisé en tant que corps qui représente un cylindre (M, R) coupé parallèlement à l'axe longitudinal qui a son plan de coupe dans l'axe longitudinal du cylindre.

3. Scalpel selon l'une quelconque des revendications précédentes, **caractérisé en ce que** la longueur de la lame (10) est dimensionnée de sorte que la lame (10) reste au sein d'une enveloppe imaginaire (50) dépassant au-delà de l'extrémité de la tête de manche (41).

4. Scalpel selon l'une quelconque des revendications précédentes, **caractérisé en ce que** la première face inclinée (45) sur la tête de manche (41) est inclinée contre l'axe longitudinal du manche (40) d'un angle (48) compris entre 25° et 35°, de préférence d'un angle de 30°.

5. Scalpel selon l'une quelconque des revendications précédentes, **caractérisé en ce que** deux tranchants (18.1, 18.2) sont réalisés sur la lame (10).

6. Scalpel selon la revendication précédente, **caractérisé en ce que** les tranchants (18.1, 18.2) de la lame (10) convergent symétriquement à l'axe longitudinal de la lame (10) sous la forme d'un triangle équilatéral (22).

7. Scalpel selon la revendication précédente, **caractérisé en ce que** la forme du triangle équilatéral se finit en un angle (22) de 45°.

8. Scalpel selon la revendication précédente, **caractérisé en ce que** les arêtes de coupe (18.1, 18.2) sont biseautées de la face arrière (14) vers le côté supérieur de lame (16).

9. Scalpel selon l'une quelconque des revendications précédentes, **caractérisé en ce que** la lame (12) se compose de céramique.

10. Scalpel selon l'une quelconque des revendications précédentes, **caractérisé en ce que** la lame (12) est collée dans la tête de manche (41).
